# EUROPÄISCHE PATENTANMELDUNG

(11) **EP 2 505 216 A1**
(43) Veröffentlichungstag der Anmeldung: **03.10.2012**
(21) Anmeldenummer: 12001829.6
(22) Anmeldetag: 19.03.2012
(51) Int. Cl.: A61M 1/00, A61B 18/00

(54) **Absaugvorrichtung von Abprodukten bei der Ablation von biologischem Gewebe**

(30) Priorität: 30.03.2011 DE 202011004532 U
(71) Anmelder: WAGNER, Justinus, 04229 Leipzig (DE)
(72) Erfinder: WAGNER, Justinus, 04229 Leipzig (DE)

(57) **Zusammenfassung**

Die vorliegende Erfindung betrifft eine Vorrichtung zur Absaugung von Abprodukten wie z.B. Rauch, Dampf, Gewebepartikel, bei der Ablation bzw. Vaporisation von biologischem Gewebe mit Hilfe eines Laserstrahles bzw. elektrochirurgischer Instrumente, bei der die Absaugvorrichtung im Sinne der Luftstromrichtung der Luftkühlung zur Sicherstellung geordneter Luftströmungsverhältnisse seitlich bzw. auf der unteren Behandlungsebene platziert ist und durch die zirkuläre Architektur eine gleichmäßig-ubiquitäre Absaugung eines Behandlungsareals gewährleistet ist. Durch zusätzliche, einzeln im Absaugvolumen einstellbare, flexible Absaugstutzen ist eine dreidimensionale Positionierung im Behandlungsraum gewährleistet.

## Beschreibung

### Technisches Gebiet

Diese Erfindung betrifft eine Vorrichtung und ein Verfahren zur Absaugung von Abprodukten, wie z.B. Rauch, Dampf, Gewebepartikel, bei der Ablation bzw. Vaporisation von biologischem Gewebe mit Hilfe eines Laserstrahles bzw. elektrochirurgischer Instrumente, insbesondere im Gesichtsbereich von Patienten.

### Stand der Technik

Thermisch-ablative Behandlungen führen zur Gewebsvaporisation und folglich zu einer störenden sowie ungesunden Rauchentwicklung. Diese Abprodukte können Schadstoffe und Krankheitserreger vorweisen und generell ein gesundheitsschädliches Potential darstellen. Eine effiziente Absaugung von Abprodukten ist demnach unabdingbar, stellt jedoch in der klinischen Anwendung meist ein Problem dar. Sperrige Absaugarme können nicht nur das Sichtfeld einschränken, sondern müssen bei ausgedehnten Gewebsablationen während der Behandlung mitgeführt - repositioniert - werden. Zudem existiert meist nur eine Absaugöffnung, auch für größere Behandlungsareale.

WO 93/16741 zeigt eine Absaugeinrichtung, die einen flexiblen Schlauch aufweist, der um die mit einer Laserstrahlvorrichtung zu behandelnde Stelle anordenbar ist und mit einer Vielzahl an beabstandeten Öffnungen versehen ist. Dieser Schlauch ist bei dieser Anmeldung in seinem Durchmesser um das Behandlungsareal variabel einstellbar.

US 5,971,977 offenbart eine Rauchschwadenabzugsvorrichtung, die um die zu operierende Stelle gelegt werden kann und entstehenden Rauch durch Öffnungen abzieht. Der Rauchabzug wird durch Perforationen, also Ausnehmungen bzw. Öffnungen, im Hauptschlauch, welcher um die operierende Stelle gelegt wird, gewährleistet.

US 5,322,521 beschreibt eine Absaugvorrichtung, welche multiple Öffnungen entlang der Längsrichtung des Schlauches offenbart. Ein Abschnitt des Schlauches wird jeweils um Körperöffnungen gelegt, während ein anderer Abschnitt dieses Schlauches in Öffnungen positioniert wird.

All diese Anordnungen ist gemeinsam, dass die Gaseintrittsöffnungen für die abzusaugende Luft durch Perforationen bzw. Öffnungen im Hauptschlauch umgesetzt wird.

DE 10 2004 021 680 A1 offenbart eine Anordnung zur Entfernung von Abprodukten bei der eine Ansaugöffnung so angeordnet werden soll, dass ein resultierender Gasstrom mit Zuhilfenahme eines Spülgases aus Ausströmungsöffnungen die Ansaugöffnung durchdringt.

### Darstellung der Erfindung

Es ist eine Aufgabe der vorliegenden Erfindung eine Vorrichtung zur Absaugung von Abprodukten wie z.B. Rauch, Dampf, Gewebepartikel, bei der Ablation bzw. Vaporisation von biologischem Gewebe mit Hilfe eines Laserstrahles bzw. elektrochirurgischer Instrumente zur Verfügung zu stellen, die eine verbesserte Handhabbarkeit aufweist und Abprodukte zuverlässig entfernen kann.

Diese Aufgabe wird mir der Vorrichtung zur Absaugung gemäß dem unabhängigen Anspruch 1 umgesetzt. Vorteilhafte Ausgestaltungen ergeben sich aus den Unteransprüchen.

Mit der erfindungsgemäßen Anordnung wird eine Vorrichtung mit kleineren Absaugstutzen (4), variabel in Zahl und Form, zur Sicherstellung einer gleichmäßigubiquitären Absaugung eines Behandlungsareals (z.B. Gesichtsareal) zur Verfügung gestellt. Zudem wird mit der erfindungsgemäßen Absaugvorrichtung das Behandlungsareal übersichtlicher. Mit der Erfindung wird eine Reduktion der Geruchsbelastung für Patient und behandelnden Arzt erreicht.

Bisherige Absaugungsvorrichtungen zeigen zumeist nach unten - entgegengesetzt des Luftkühlungsstromes, der generell vom Laserhandstück bzw. eigenständig von oben/seitlich austritt - und saugen die von der Kühlungsluft verwirbelten, zur Seite bzw. nach unten gelenkten Abprodukte in Gegenrichtung dieses Luftkühlungsstromes auf. Demnach "entweicht" der Großteil der Kühlluft mit den Abprodukten meist in den Raum.

Der durch die Erfindung erreichte Fortschritt bzw. Vorteil besteht u.a. in der Anordnung der Absaugstutzen (4) an der Seite bzw. bodennah, welche in Richtung des zugeführten Luftstromes zeigen und somit den Luftstrom nicht "knicken" (6). Diese im Raum frei positionierbaren Absaugstutzen, welche überdies durch Ventile einzeln in ihrer Absaugleistung eingestellt werden können, gewährleisten eine dreidimensionale Absaugungsanordnung im Behandlungsareal. Die Kühlvorrichtungsaustrittsöffnung wird mit der Absaugungsöffnung physikalisch über den Luftstrom "verbunden" und stellt in diesem Sinn ein kommunizierendes System dar. Dies äußert sich in einer effektiven Absaugung, da die meisten Abprodukte - auch bei geringerer Leistung des Kühlgerätes bzw. des Absauggerätes - mittels direkter Luftströmungsrichtung (6) und bei geringer Absaugungsstrecke zugeführt und aufgenommen werden.

Überdies ist eine effiziente Oberflächen-/bzw. Hautkühlung bei thermischen Ablationen von medizinischer Wichtigkeit, da es ansonsten als Maximalvariante zu Verbrennung des Gewebes kommen kann. Bisher ist der Kühlungseffekt hauptsächlich von der apparativen Leistung des Kühlgerätes abhängig.

Der Erfindung liegt zudem die Aufgabe zugrunde, durch die fortführende Anordnung des Luftströmungsflusses zwischen Kühlluftaustrittsöffnung und Absaugungsöffnung (6) mithilfe eines Windkühlungseffekts das Gewebe je nach Luftströmungsgeschwindigkeit zusätzlich abzukühlen.

Diese Vorrichtung stellt zum Zweck der Sicherstellung geordneter Luftströmungsverhältnisse eine Neuentwicklung dar.

Diese Vorrichtung ist ein "Bindeglied" zwischen herkömmlichen Absaugungen und Kühlungen, kann jedoch auch ohne zuführende Kühlung allein als Absaugungszubehör eingesetzt werden. Mit jeweils unterschiedlichen Konnektoren (1) kann diese Absaugvorrichtung an handelsübliche Absaugrohre/-vorrichtungen angeschlossen werden.

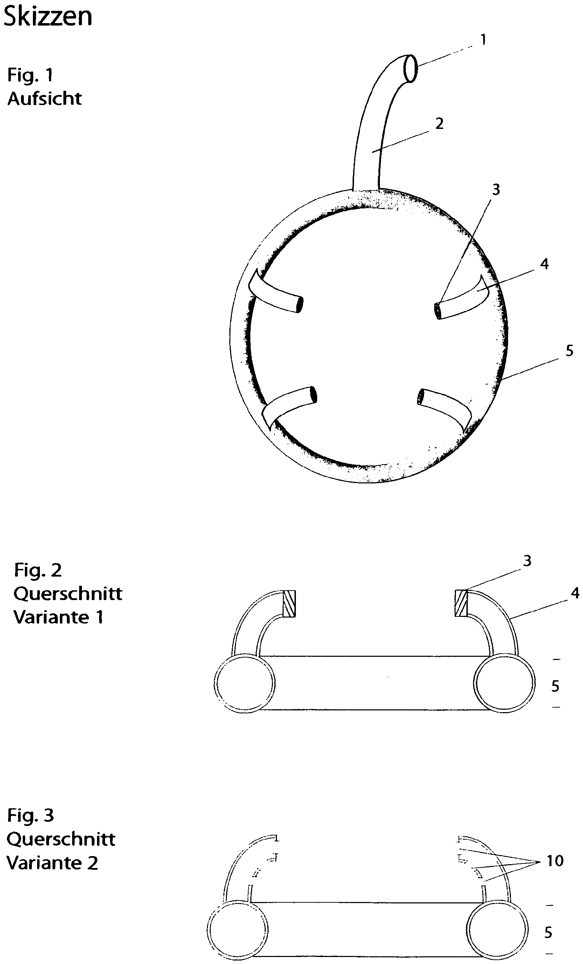

Kurze Beschreibung der Zeichnungen
- Fig. 1: zeigt eine Aufsicht auf eine erfindungsgemäße Absaugvorrichtung,
- Fig. 2: zeigt eine erste Variante einer erfindungsgemäßen Absaugvorrichtung im Querschnitt,
- Fig. 3: zeigt eine zweite Variante einer erfindungsgemäßen Absaugvorrichtung im Querschnitt, und

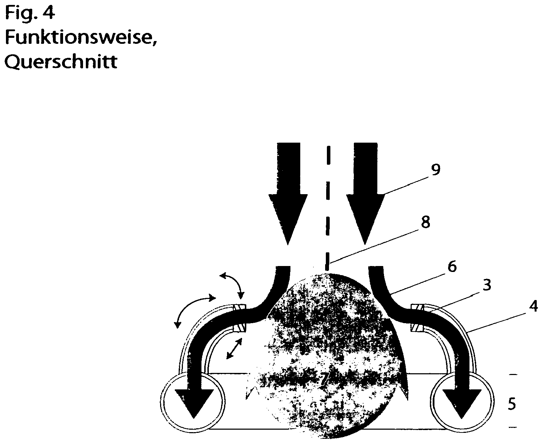

- Fig. 4: erläutert die Funktionsweise einer erfindungsgemäßen Absaugvorrichtung.

### Ausführliche Beschreibung bevorzugter Ausführungsformen

Im Folgenden erfolgt eine rein beispielhafte Beschreibung einer erfindungsgemäßen Absaugvorrichtung anhand bevorzugter Ausführungsbeispiele. Alternativen zu dem beschriebenen Ausführungsbeispiel sind möglich.

Die Absaugungsvorrichtung besteht hier beispielhaft aus einem zirkulären Masterschlauch 5, kann jedoch eine beliebige Gestalt und Form im Sinne der Funktion aufweisen. In anderen Worten kann der Masterschlauch 5 in Draufsicht eine im Wesentlichen runde Form oder jede andere Form aufweisen, die zur Sicherstellung einer ausreichenden Absaugung ausreichend ist. Selbiges gilt für die Querschnittsform des Masterschlauches im Inneren. An diesem beispielhaften zirkulären Grundschlauch 5 sind hier beispielhaft in vier Quadranten angebrachte Absaugstutzen 4, welche ihrerseits flexibel in Form, Länge, Positionierung, Absaugvolumen und Anzahl sind. Die Flexibilität der Absaugstutzen 4 wird durch ein biegsames, formstabiles Material erreicht, welches in der Länge variabel und in der Positionierung im Raum frei einstellbar ist. Die einströmenden Luftvolumina können durch optionale Ventile 3 an den Enden der Absaugstutzen 4 eingestellt werden. Somit sind die Absaugvolumina der einzelnen Absaugstutzen 4 einstellbar. Die Verbindung zwischen Masterschlauch 5 und handelsüblichen Sauggeräten wird mittels individueller Konnektoren 1 gewährleistet. Der Kühlungsluftstrom 9 wird hier beispielhaft vom Kühlgerät über das zuführende Laserhandstück gewährleistet.

Die Realisierung der Erfindung ist nicht an das dargestellte Ausführungsbeispiel gebunden, fachmännische Weiterentwicklungen führen nicht zu einem Verlassen des Schutzbereiches.

### Bezugszeichenliste:

- 1: Konnektor
- 2: Verbindungsstück zw. Konnektor und Masterschlauch
- 3: einstellbares Ventil an der Absaugöffnung zur Regulation des Saugvolumens
- 4: Absaugstutzen, flexibel in Länge und in allen Richtungen frei positionierbar
- 5: Masterschlauch
- 6: Luftströmungsverlauf mit Windkühlungseffekt am Gewebe
- 7: Patient (als Bsp.)
- 8: Laserstrahl (als Bsp.)
- 9: Kühlluftstrom der Kühleinheit (als Bsp.)
- 10: Absaugöffnungen, multiple, im Durchmesser zum Ende hin zunehmend

## Patentansprüche

1. Vorrichtung zur Absaugung von Abprodukten wie z.B. Rauch, Dampf, Gewebepartikel, bei der Ablation bzw. Vaporisation von biologischem Gewebe mit Hilfe eines Laserstrahles bzw. elektrochirurgischer Instrumente, bei der die Absaugvorrichtung im Sinne der Luftstromrichtung der Luftkühlung zur Sicherstellung geordneter Luftströmungsverhältnisse seitlich bzw. auf der unteren Behandlungsebene platziert ist und durch die zirkuläre Architektur eine gleichmäßig-ubiquitäre Absaugung eines Behandlungsareals gewährleistet ist,
**dadurch gekennzeichnet, dass** eine variable Anzahl von Absaugstutzen (4), ihrerseits flexibel in Form, Länge, Positionierung und Absaugvolumen, vorhanden sind.

2. Vorrichtung nach Anspruch 1, **dadurch gekennzeichnet, dass** jeder einzelne Absaugstutzen (4) jeweils ein Ventil (10) aufweisen kann und das Luftvolumen an den Enden der Absaugstutzen eingestellt werden kann.

3. Vorrichtung nach Anspruch 1 oder 2, **dadurch gekennzeichnet, dass** die Absaugstutzenarchitektur Absaugöffnungen am Ende des Absaugstutzens einzeln und/oder mehrere Absaugöffnungen (10) längs über die Länge des Absaugstutzens (4) verteilt aufweist.

4. Vorrichtung nach Anspruch 3, **dadurch gekennzeichnet, dass** bei multiplen längs versehenen Absaugungsöffnungen (10) am Absaugstutzen (4) diese zum Ende hin im Durchmesser zunehmen.

5. Vorrichtung nach einem der vorgenannten Ansprüche, **dadurch gekennzeichnet, dass** eine zusätzliche Oberflächen-/bzw. Hautkühlung durch die fortführende Anordnung des Luftströmungsflusses zwischen Kühlluftaustrittsöffnung und Absaugungsöffnung mittels "Windkühlungseffekt" unterstützt wird.
